# EUROPEAN PATENT SPECIFICATION

(11) **EP 2 880 571 B1**
(45) Date of publication and mention of the grant of the patent: **17.07.2019**
(21) Application number: 13763302.0
(22) Date of filing: 01.08.2013
(51) Int. Cl.: G16H 10/00

(54) **METHOD AND DEVICE FOR GENERATING AN EXERCISE PLAN**
VERFAHREN UND VORRICHTUNG ZUR ERZEUGUNG EINES ÜBUNGSPLANS
PROCÉDÉ ET DISPOSITIF POUR GÉNÉRER UN PLAN D'EXERCICE

(30) Priority: 06.08.2012 WO PCT/CN2012/001039
(43) Date of publication of application: 10.06.2015
(73) Proprietor: Koninklijke Philips N.V., 5656 AE Eindhoven (NL)
(72) Inventor: LIN, Hong, NL-5656 AE Eindhoven (NL); WU, Haonan, NL-5656 AE Eindhoven (NL)
(74) Representative: Steffen, Thomas
(86) International application number: PCT/IB2013/056319
(87) International publication number: WO 2014/024103

(56) References cited:
- WO-A1-2010/041000
- US-A1- 2007 219 059

## Description

### FIELD OF THE INVENTION

The invention relates to generation of an exercise plan, and more specifically to method and device for generating an exercise plan from a plurality of exercises for a user.

### BACKGROUND OF THE INVENTION

Serious illnesses such as a stroke, or surgical operations such as the insertion of joint implants may be the cause of disability of a user. For example, after a stroke, the region of the brain responsible for controlling a muscle or a number of muscles in a functional chain, may be damaged. Thus, the affected muscles degenerate. The user should do exercises to train the affect muscles and joints in order to, at least partially, restore the lost functions. WO 2010/041000 discloses a system and method for improving the fitness of an individual based on assigning the individual a weekly points value reflective of an amount of activity that the individual needs to undertake to maintain a certain level of fitness.

It has been proposed to solve the problem of how to determine the proper plurality of exercises to be done by the user according to his current health state. In practice, however, the determined plurality of exercises is always too many for the user to do in one session. In this respect, no relative processing concerns with how to generate an exercise plan from the determined exercises for the user or how to update the exercise plan.

### SUMMARY OF THE INVENTION

It would be desirable to obviate at least some of the above disadvantages and provide a method and device for generating an exercise plan from a plurality of exercises for a user.

To better address one or more of these concerns, the invention is as defined in and by the appended claims.

There is also described a method for generating an exercise plan from a plurality of exercises for a user is proposed. Each exercise of the plurality of exercises is associated with a parameter for indicating the status of said each exercise of the plurality of exercises. The parameter can be set to different value. For example, when the parameter is set to a first value, it indicates that the associated exercise has not been selected to be part of the exercise plan; similarly, a second value indicates that the associated exercise has been selected to be part of the exercise plan; and a third value indicates that the associated exercise has been performed by the user. The method comprises the steps of: a) initializing the parameter of each exercise of the plurality of exercises into the first value; b) selecting a subset of exercises from the plurality of exercises to generate the exercise plan and updating the associated parameter of each selected exercise in the plurality of exercises into the second value; and c) updating the exercise plan based on the user's performance resulting from the execution of the exercise plan and the parameter associated with each exercise of the plurality of exercises.

Due to the above method, an exercise plan can be generated from a plurality of exercises for a user and the generated exercise plan can be updated conveniently.

The step c) of the method comprises: c1) deleting at least one exercise from the exercise plan based on the user's performance resulting from the execution of the exercise plan and updating the parameter of each deleted exercise in the plurality of exercises into the third value; and c2) adding at least one exercise with the first value from the plurality of exercises into the exercise plan and updating the parameter of each added exercise in the plurality of exercises into the second value.

The step c) can further comprise a step c3) before the step c2): determining whether the number of the exercises with the first value is larger than or equal to the difference between the number of the exercises in the subset and the number of the exercises with the second value. When the step c3) is comprised, the addition of at least one exercise with the first value from the plurality of exercises into the exercise plan in the step c2) can comprise: if the number of the exercises with the first value is larger than or equal to the difference between the number of the exercises in the subset and the number of the exercises with the second value, adding at least one exercise with the first value from the plurality of exercises into the exercise plan so that the sum of the number of the added exercise and the number of the exercises with the second value is equal to the number of the exercises in the subset; or, if the number of the exercises with the first value is not larger than or equal to the difference between the number of the exercises in the subset and the number of the exercises with the second value, adding all the exercises with the first value from the plurality of exercises into the exercise plan.

The step c) further comprises a step c4) after the step c2): determining whether a predefined condition is met, and updating the parameter of each of all the exercises with the third value into the first value if the predefined condition is met. For example, the predefined condition can be that the number of the exercises with the first value is less than half of the number of the exercises in the subset.

The method can further comprise returning to the step c1) after the step c4) is performed and repeating the steps c1), c3), c2) and c4) at least one time.

There is also described a device for generating an exercise plan from a plurality of exercises for a user is proposed. Each exercise of the plurality of exercises is associated with a parameter for indicating the status of said each exercise of the plurality of exercises. The parameter can be set to different value. For example, when the parameter is set to a first value, it may indicate that the associated exercise has not been selected to be part of the exercise plan; similarly, a second value may indicate that the associated exercise has been selected to be part of the exercise plan; and a third value may indicate that the associated exercise has been performed by the user. The device comprises: a first unit, configured to initialize the parameter of each exercise of the plurality of exercises into the first value; a second unit, configured to select a subset of exercises from the plurality of exercises having been initialized to generate the exercise plan and update the parameter of each selected exercise in the plurality of exercises into the second value; and a third unit, configured to update the exercise plan based on the user's performance resulting from the execution of the exercise plan and the parameter associated with each exercise of the plurality of exercises.

The third unit comprises: a first subunit, configured to delete at least one exercise from the exercise plan based on the user's performance resulting from the execution of the exercise plan and update the parameter of each deleted exercise in the plurality of exercises into the third value; and a second subunit, configured to add at least one exercise with the first value from the plurality of exercises into the exercise plan from which at least one exercise has been deleted and update the parameter of each added exercise in the plurality of exercises into the second value.

The third unit can further comprise a third subunit, which is configured to, for the plurality of exercises in which the parameter of each deleted exercise deleted from the exercise plan has been updated into the third value, determining whether the number of the exercises with the first value is larger than or equal to the difference between the number of the exercises in the subset and the number of the exercises with the second value. When the third subunit is comprised, the second subunit can be further configured to: if the number of the exercises with the first value is larger than or equal to the difference between the number of the exercises in the subset and the number of the exercises with the second value, add at least one exercise with the first value from the plurality of exercises into the exercise plan from which at least one exercise has been deleted so that the sum of the number of the added exercise and the number of the exercises with the second value is equal to the number of the exercises in the subset; or, if the number of the exercises with the first value is not larger than or equal to the difference between the number of the exercises in the subset and the number of the exercises with the second value, add all the exercises with the first value from the plurality of exercises into the exercise plan from which at least one exercise has been deleted.

The third unit further comprises a fourth subunit, which is configured to, for the plurality of exercises in which the parameter of each added exercise added into the exercise plan has been updated into the second value, determine whether a predefined condition is met and update the parameter of each of all the exercises with the third value into the first value if the predefined condition is met. For example, the predefined condition can be that the number of the exercises with the first value is less than half of the number of the exercises in the subset.

The operations of the first subunit, the third subunit, the second subunit and the fourth subunit can be repeated at least one time.

There is also described an information medium, which is configured to store a program loaded and executed by a data processing device to perform the described method(s).

These and other aspects will be apparent from and elucidated with reference to the examples described hereinafter.

### BRIEF DESCRIPTION OF THE DRAWINGS

The proposed method and device for generating an exercise plan from a plurality of exercises for a user will be described in the following by way of examples and with reference to the accompanying drawings, without limiting the scope of protection as defined by the claims. In the Figures:
Figure 1 schematically shows a flow chart of a method for generating an exercise plan from a plurality of exercises for a user according to an example;
Figure 2 schematically shows a flow chart of a method for generating an exercise plan from a plurality of exercises for a user according to another example;
Figure 3 schematically shows a flow chart of a method for generating an exercise plan from a plurality of exercises for a user according to a further example;
Figure 4 is a schematic view of a device for generating an exercise plan from a plurality of exercises for a user according to an example;
Figure 5 is a schematic view of a device for generating an exercise plan from a plurality of exercises for a user according to another example; and
Figure 6 is a schematic view of a device for generating an exercise plan from a plurality of exercises for a user according to a further example.

### DETAILED DESCRIPTION OF EMBODIMENTS

While the invention covers various modifications and alternative constructions, examples are shown in the drawings and will hereinafter be described in detail. However, it should be understood that the specific description and drawings are not intended to limit the invention to the specific forms disclosed. On the contrary, it is intended that the scope of the claimed invention includes all modifications and alternative constructions thereof falling within the scope of the invention as expressed in the appended claims.

As described above, for a user, such as a patient to be rehabilitated, it may be desirable to generate an exercise plan from a plurality of exercises for him/her and/or to update the exercise plan. However, no relative processing focuses on this respect currently. The inventor has recognized this situation and proposed a mechanism to address this issue.

Suppose the exercise plan can be generated from a plurality of exercises which are determined in advance. To generate and update the exercise plan conveniently, the inventor has proposed to make each exercise of the plurality of exercises associated with a parameter. For example, the parameter can be set to different value for indicating the different status of the exercise. For example, a first value first value indicates that the associated exercise has not been selected to be part of the exercise plan, i.e. the first value is associated with an "Unselected" status of the exercise; a second value indicates that the associated exercise has been selected to be part of the exercise plan, i.e. the second value is associated with a "Selected" status of the exercise; and a third value indicates that the associated exercise has been performed by the user, i.e. the third value is associated with a "Done" status of the exercise. When the plurality of exercises is initialized, the parameter of each exercise of the plurality of exercises is initialized into the first value. Then, some of exercises are selected from the plurality of exercises to generate the exercise plan, and the parameter of each selected exercise in the plurality of exercises is updated into the second value. After the user finishes doing the exercise plan and gets back with exercise performance, the exercise plan can be updated based on the user's performance and the parameter associated with each exercise of the plurality of exercises.

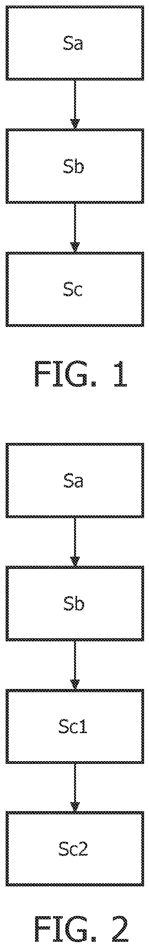

Figure 1 schematically shows a flow chart of a method for generating an exercise plan from a plurality of exercises for a user according to an example.

Each exercise of the plurality of exercises can be associated with a parameter for indicating the status of said each exercise of the plurality of exercises. The parameter can be set to the first value for indicating that the associated exercise has not been selected to be part of the exercise plan; or can be set to the second value for indicating that the associated exercise has been selected to be part of the exercise plan; or can be set to the third value for indicating that the associated exercise has been performed by the user. As shown in Figure 1, the method can comprise at least the following steps: at step Sa, the parameter of each exercise of the plurality of exercises is initialized into the first value; at step Sb, a subset of exercises is selected from the plurality of exercises to generate the exercise plan and the parameter of each selected exercise in the plurality of exercises is updated into the second value; and at step Sc, the exercise plan is updated based on the user's performance resulting from the execution of the exercise plan and the parameter associated with each exercise of the plurality of exercises.

Figure 2 schematically shows a flow chart of a method for generating an exercise plan from a plurality of exercises for a user according to another example.

For clarity and simplicity, for the steps identical to those in the method as shown in Figure 1, no further explanation will be provided.

As shown in Figure 2, the method for generating an exercise plan from a plurality of exercises for a user according to this example can comprise steps Sa and Sb which are identical to those in the method as shown in Figure 1. Furthermore, the step Sc in the method according to this example can comprise at least two substeps: at step Sc1, at least one exercise from the exercise plan is deleted based on the user's performance resulting from the execution of the exercise plan and the parameter of each deleted exercise in the plurality of exercises is updated into the third value; and at step Sc2, at least one exercise with the first value is added from the plurality of exercises into the exercise plan and the parameter of each added exercise in the plurality of exercises is updated into the second value.

In the method as shown in Figure 2, the exercise plan can be updated by deleting at least one exercise from the exercise plan based on the user's performance and adding at least one exercise with "Unselected" status into the exercise plan.

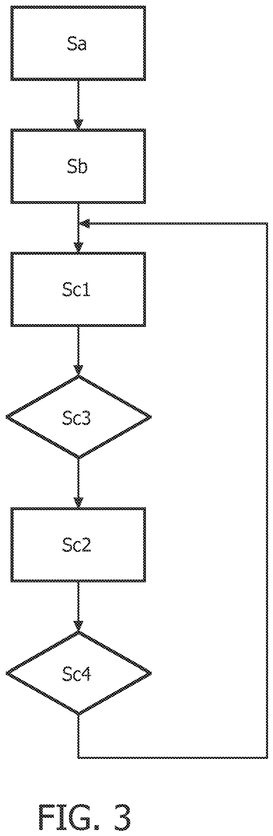

Figure 3 schematically shows a flow chart of a method for generating an exercise plan from a plurality of exercises for a user according to a further example.

For clarity and simplicity, for the steps identical to those in the methods as shown in Figure 2, no further explanation will be provided.

As shown in Figure 3, the method for generating an exercise plan from a plurality of exercises for a user according to this embodiment can comprise steps Sa, Sb, Sc1 and Sc2 which are identical to those in the method as shown in Figure 2. Furthermore, the step Sc in the method according to this embodiment can further comprise an optional substep Sc3 before the substep Sc2. At the step Sc3, whether the number of the exercises with the first value is larger than or equal to the difference between the number of the exercises in the subset and the number of the exercises with the second value is determined.

If the substep Sc3 is performed, the addition of at least one exercise with the first value from the plurality of exercises into the exercise plan in the substep Sc2 can comprise: if the number of the exercises with the first value is larger than or equal to the difference between the number of the exercises in the subset and the number of the exercises with the second value, at least one exercise with the first value is added from the plurality of exercises into the exercise plan so that the sum of the number of the added exercise and the number of the exercises with the second value is equal to the number of the exercises in the subset; or, if the number of the exercises with the first value is not larger than or equal to the difference between the number of the exercises in the subset and the number of the exercises with the second value, all the exercises with the first value are added from the plurality of exercises into the exercise plan.

Due to the substeps Sc3 and Sc2, it is possible to keep a desired number of exercises in the exercise plan.

Optionally, the step Sc in the method according to this embodiment can further comprise a substep Sc4 after the substep Sc2. At the step Sc4, whether a predefined condition is met is determined and the parameter of each of all the exercises with the third value is updated into the first value if the predefined condition is met.

In practice, the predefined condition can be set or determined according to applications and/or requirements. For example, the predefined condition can be that the number of the exercises with the first value is less than half of the number of the exercises in the subset. But it should be understood that the specific example is not intended to limit the scope of the invention.

In some cases, it may be desirable to keep updating the exercise plan. To address this concern, optionally, the method can further comprise returning to the step c1) after the step c4) is performed and repeating the steps c1), c3), c2) and c4) at least one time, as shown in Figure 3.

Due to the optional step Sc4, the parameters of all the exercises with the third value can be updated into the first value (from "Done" status into "Unselected" status) when there are less exercises with the first value ("Unselected" status) existing in the plurality of exercises. Therefore, it is possible to continue doing the update of the exercise plan.

In practice, there are many ways for evaluating the user's performance. The following Table 1 is merely an example of evaluating the user's performance. It should be understood that the specific example is not intended to limit the scope of the invention. A skilled person can determine or select the way for evaluating the user's performance according to applications and/or requirements.

**Table 1**

| Error | Description | Evaluation |
|---|---|---|
| Not Reach the Range | Whether the user can reach the target exercise need | Good: < 30% repetition exercise cannot reach target |
| | | Poor: ≥ 30% repetition exercise cannot reach target |
| Trunk Move | Whether trunk move happened during the user does exercise | Good: < 20% repetition exercise with trunk move |
| | | Poor: ≥ 20% repetition exercise with trunk move |
| Speed Control | Whether the user do well on speed control | Good: < 20% repetition exercise with speed is too fast |
| | | Poor: ≥ 20% repetition exercise with speed is too fast |
| Elbow Move | Whether elbow move happened during the user does exercise | Good: < 20% repetition exercise with elbow move |
| | | Poor: ≥ 20% repetition exercise with elbow move |

As an example, suppose each exercise needs to be practiced with several repetitions by the user. And each repetition has four attributes to show whether the user does the exercise with some error or not. Finally all attributes of all the repetitions can be summarized to only two kinds of results: Good and Poor. If any error item gets Poor performance, the exercise summary performance is Poor; else the exercise summary performance is Good. The detail information of attributes can be got in the above Table 1.

As described above, in the method(s) for generating an exercise plan from a plurality of exercises for a user according to embodiment(s) of the present invention, the exercise plan can be updated based on the user's performance. For example, if the above Table 1 is used for evaluating the user's performance, the good performed exercise(s) can be deleted from the exercise plan and the poor performed exercise(s) can be remained in the exercise plan. In another example, the good performed exercise(s) can be deleted from the exercise plan, while for the poor performed exercise(s), there are two situations: if the poor performance results from any one of the reasons of Trunk Move, Elbow Move and Speed control, such poor performed exercise(s) can be deleted from the exercise plan; or else, if the poor performance results from other reason, such poor performed exercise(s) can be remained in the exercise plan.

Some or all of the steps of the methods as described above may alternatively be implemented by a data processing device in combination with e.g. a computer program. The computer program comprises computer readable code which when loaded and executed by the data processing device, enables at least some steps of any of the methods described herein to be performed. The computer program may be stored on an information medium.

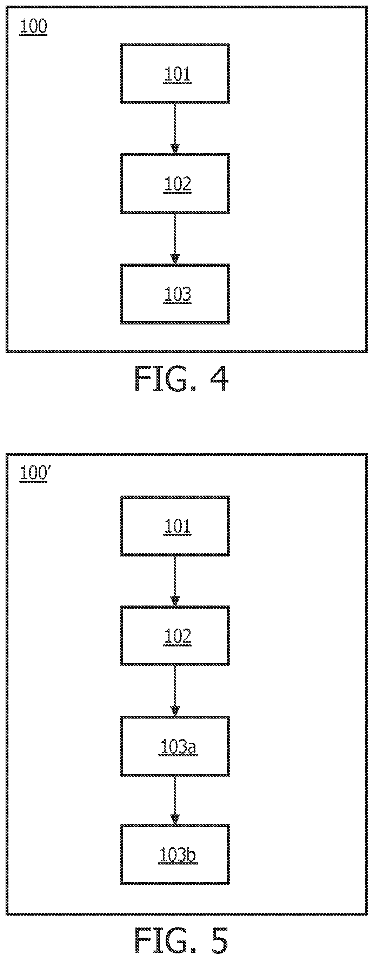

Figure 4 is a schematic view of a device 100 for generating an exercise plan from a plurality of exercises for a user according to an example.

Each exercise of the plurality of exercises can be associated with a parameter for indicating the status of said each exercise of the plurality of exercises. The first value indicates that the associated exercise has not been selected to be part of the exercise plan; the second value indicates that the associated exercise has been selected to be part of the exercise plan; and the third value indicates that the associated exercise has been performed by the user. As shown in Figure 4, the device 100 can comprise: a first unit 101, configured to initialize the parameter of each exercise of the plurality of exercises into the first value; a second unit 102, configured to select a subset of exercises from the plurality of exercises having been initialized to generate the exercise plan and update the parameter of each selected exercise in the plurality of exercises into the second value; and a third unit 103, configured to update the exercise plan based on the user's performance resulting from the execution of the exercise plan and the parameter associated with each exercise of the plurality of exercises.

Here, the device 100 is elucidated by way of units, while in practice, the device 100 can be implemented by way of either software, hardware or a combination thereof. For example, program codes achieving the functions of the above units can be stored in a memory. These codes can be loaded and executed by a processor in a computer to implement some or all of the functionality of all units of the device 100. In another example, special hardware circuits such as Application Specific Integrated Circuits (ASICs) or digital signal processor (DSP) may be used in practice to implement some or all of the functionality of all units of the device 100. For instance, certain IC chips can achieve the functions of the above units, and these chips can be controlled by an MCU to implement some or all of the functionality of all units of the device 100.

Figure 5 is a schematic view of a device 100' for generating an exercise plan from a plurality of exercises for a user according to another example.

For clarity and simplicity, for the units identical to those in the device as shown in Figure 4, no further explanation will be provided.

As shown in Figure 5, the device 100' for generating an exercise plan from a plurality of exercises for a user according to this example can comprise a first unit 101 and a second unit 102 which are identical to those in the device as shown in Figure 4. Furthermore, the third unit in the device according to this example can comprise at least two subunits: a first subunit 103a, which is configured to delete at least one exercise from the exercise plan based on the user's performance resulting from the execution of the exercise plan and update the parameter of each deleted exercise in the plurality of exercises into the third value; and a second subunit 103b, which is configured to add at least one exercise with the first value from the plurality of exercises into the exercise plan from which at least one exercise has been deleted and update the parameter of each added exercise in the plurality of exercises into the second value.

Here, the device 100' is elucidated by way of units, while in practice, the device 100' can be implemented by way of either software, hardware or a combination thereof. For example, program codes achieving the functions of the above units can be stored in a memory. These codes can be loaded and executed by a processor in a computer to implement some or all of the functionality of all units of the device 100'. In another example, special hardware circuits such as Application Specific Integrated Circuits (ASICs) or digital signal processor (DSP) may be used in practice to implement some or all of the functionality of all units of the device 100'. For instance, certain IC chips can achieve the functions of the above units, and these chips can be controlled by an MCU to implement some or all of the functionality of all units of the device 100'.

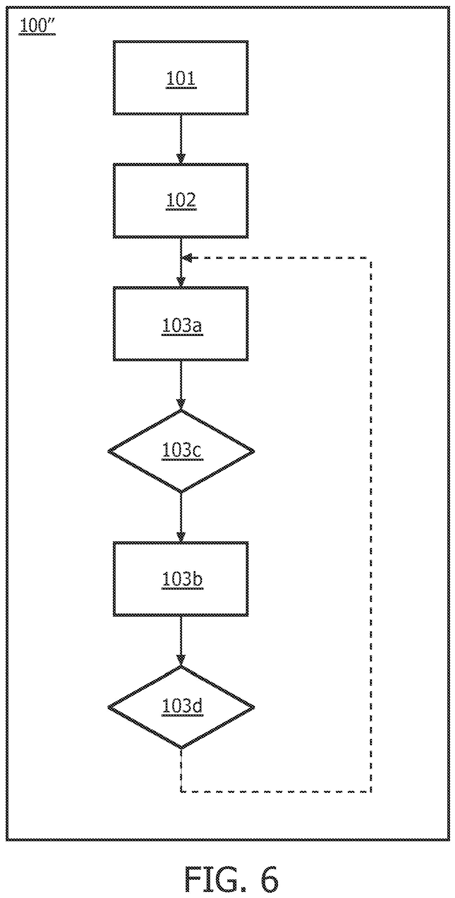

Figure 6 is a schematic view of a device 100" for generating an exercise plan from a plurality of exercises for a user according to an embodiment of the present invention.

For clarity and simplicity, for the units or subunits identical to those in the device as shown in Figure 5, no further explanation will be provided.

As shown in Figure 6, the device 100" for generating an exercise plan from a plurality of exercises for a user according to this embodiment comprises a first unit 101, a second unit 102, a first subunit 103a of a third unit and a second subunit 103b of the third unit, which are identical to those in the device as shown in Figure 5. Furthermore, the third unit in the device 100" according to another embodiment can further comprise an optional third subunit 103c, which is configured to, for the plurality of exercises in which the parameter of each deleted exercise deleted from the exercise plan has been updated into the third value, determining whether the number of the exercises with the first value is larger than or equal to the difference between the number of the exercises in the subset and the number of the exercises with the second value.

When the device 100" comprises the third subunit 103c, the second subunit 103b can be further configured to: if the number of the exercises with the first value is larger than or equal to the difference between the number of the exercises in the subset and the number of the exercises with the second value, add at least one exercise with the first value from the plurality of exercises into the exercise plan from which at least one exercise has been deleted so that the sum of the number of the added exercise and the number of the exercises with the second value is equal to the number of the exercises in the subset; or, if the number of the exercises with the first value is not larger than or equal to the difference between the number of the exercises in the subset and the number of the exercises with the second value, add all the exercises with the first value from the plurality of exercises into the exercise plan from which at least one exercise has been deleted.

The third unit in the device 100" according to this embodiment further comprises a fourth subunit 103d, which is configured to, for the plurality of exercises in which the parameter of each added exercise added into the exercise plan has been updated into the second value, determine whether a predefined condition is met and update the parameter of each of all the exercises with the third value into the first value if the predefined condition is met.

In practice, the predefined condition can be set or determined according to applications and/or requirements. For example, the predefined condition can be that the number of the exercises with the first value is less than half of the number of the exercises in the subset. But it should be understood that the specific example is not intended to limit the scope of the invention.

In some cases, it may be desirable to keep updating the exercise plan. To address this concern, optionally, the operations of the first subunit 103a, the third subunit 103c, the second subunit 103b and the fourth subunit 103d can be repeated at least one time, as shown in Figure 6.

Here, the device 100" is elucidated by way of units, while in practice, the device 100" can be implemented by way of either software, hardware or a combination thereof. For example, program codes achieving the functions of the above units can be stored in a memory. These codes can be loaded and executed by a processor in a computer to implement some or all of the functionality of all units of the device 100". For instance, certain IC chips can achieve the functions of the above units, and these chips can be controlled by an MCU to implement some or all of the functionality of all units of the device 100".

It should be understood that, although the present invention can be applied for generating an exercise plan for rehabilitation of a user, such as a patient, it is not intended to limit the present invention into such application. For example, the present invention can also be used for generating an exercise plan for training in sports or for losing weight. These and other applications still fall within the scope of the present invention.

## Claims

1. A computer-implemented method for generating an exercise plan from a plurality of exercises for a user, each exercise of the plurality of exercises being associated with a parameter for indicating the status of said each exercise of the plurality of exercises, the method comprising the steps of:
a) initializing the parameter of each exercise of the plurality of exercises into a first value for indicating that the associated exercise has not been selected to be part of the exercise plan;
b) selecting a subset of exercises from the plurality of exercises to generate the exercise plan and updating the associated parameter of each selected exercise in the plurality of exercises into a second value for indicating said each selected exercise has been selected to be part of the exercise plan; and
c) after the user finishes doing the exercise plan, updating the exercise plan based on the user's performance resulting from the execution of the exercise plan and the parameter associated with each exercise of the plurality of exercises, wherein the step c) comprises:
c1) deleting at least one exercise from the exercise plan based on the user's performance resulting from the execution of the exercise plan and updating the parameter of each deleted exercise in the plurality of exercises into a third value for indicating said each deleted exercise has been performed by the user; and
c2) adding at least one exercise with the first value from the plurality of exercises into the exercise plan and updating the parameter of each added exercise in the plurality of exercises into the second value, and
wherein the step c) further comprises a step c4) after the step c2): determining whether a predefined condition is met, and updating the parameter of each of the exercises with the third value into the first value if the predefined condition is met.

2. A method according to claim 1, wherein the step c) further comprises a step c3) before the step c2): determining whether the number of the exercises with the first value is larger than or equal to the difference between the number of the exercises in the subset and the number of the exercises with the second value.

3. A method according to claim 2, wherein the addition of at least one exercise with the first value from the plurality of exercises into the exercise plan in the step c2) comprises:
if the number of the exercises with the first value is larger than or equal to the difference between the number of the exercises in the subset and the number of the exercises with the second value, adding at least one exercise with the first value from the plurality of exercises into the exercise plan so that the sum of the number of the added exercise and the number of the exercises with the second value is equal to the number of the exercises in the subset; or
if the number of the exercises with the first value is not larger than or equal to the difference between the number of the exercises in the subset and the number of the exercises with the second value, adding all the exercises with the first value from the plurality of exercises into the exercise plan.

4. A method according to claim 1, wherein the predefined condition is that the number of the exercises with the first value is less than half of the number of the exercises in the subset.

5. A method according to claim 1, further comprising returning to the step c1) after the step c4) is performed and repeating the steps c1), c3), c2) and c4) at least one time.

6. A device (100, 100', 100") for generating an exercise plan from a plurality of exercises for a user, each exercise of the plurality of exercises being associated with a parameter for indicating the status of said each exercise of the plurality of exercises, the device comprising:
a first unit (101), configured to initialize the parameter of each exercise of the plurality of exercises into a first value for indicating that the associated exercise has not been selected to be part of the exercise plan;
a second unit (102), configured to select a subset of exercises from the plurality of exercises having been initialized to generate the exercise plan and update the associated parameter of each selected exercise in the plurality of exercises into a second value for indicating said each exercise in the subset has been selected to be part of the exercise plan; and
a third unit (103), configured to, after the user finishes doing the exercise plan, update the exercise plan based on the user's performance resulting from the execution of the exercise plan and the parameter associated with each exercise of the plurality of exercises,
wherein the third unit comprises:
a first subunit (103a), configured to delete at least one exercise from the exercise plan based on the user's performance resulting from the execution of the exercise plan and update the parameter of each deleted exercise in the plurality of exercises into a third value for indicating said each deleted exercise has been performed by the user; and
a second subunit (103b), configured to add at least one exercise with the first value from the plurality of exercises into the exercise plan from which at least one exercise has been deleted and update the parameter of each added exercise in the plurality of exercises into the second value, and
wherein the third unit (103) further comprises a fourth subunit (103d), configured to, for the plurality of exercises in which the parameter of each added exercise added into the exercise plan has been updated into the second value, determine whether a predefined condition is met and update the parameter of each of the exercises with the third value into the first value if the predefined condition is met.

7. A device according to claim 6, wherein the third unit further comprises a third subunit (103c), configured to, for the plurality of exercises in which the parameter of each deleted exercise deleted from the exercise plan has been updated into the third value, determine whether the number of the exercises with the first value is larger than or equal to the difference between the number of the exercises in the subset and the number of the exercises with the second value.

8. A device according to claim 7, wherein the second subunit (103b) is further configured to:
if the number of the exercises with the first value is larger than or equal to the difference between the number of the exercises in the subset and the number of the exercises with the second value, add at least one exercise with the first value from the plurality of exercises into the exercise plan from which at least one exercise has been deleted so that the sum of the number of the added exercise and the number of the exercises with the second value is equal to the number of the exercises in the subset; or
if the number of the exercises with the first value is not larger than or equal to the difference between the number of the exercises in the subset and the number of the exercises with the second value, add all the exercises with the first value from the plurality of exercises into the exercise plan from which at least one exercise has been deleted.

9. A device according to claim 6, wherein the predefined condition is that the number of the exercises with the first value is less than half of the number of the exercises in the subset.

10. A device according to claim 6, wherein the operations of the first subunit (103a), the third subunit (103c), the second subunit (103b) and the fourth subunit (103d) are repeated at least one time.

11. An information medium, configured to store a program loaded and executed by a processor in a computer to perform a method according to any one of claims 1 to 5.

## Patentansprüche

1. Computerimplementiertes Verfahren zum Erzeugen eines Übungsplans aus einer Vielzahl von Übungen für einen Benutzer, wobei jede Übung aus der Vielzahl von Übungen mit einem Parameter verknüpft ist, um den Status der/jeder Übung aus der Vielzahl von Übungen anzugeben, wobei das Verfahren die Schritte umfasst des:
a) Initialisierens des Parameters jeder Übung aus der Vielzahl von Übungen mit einem ersten Wert, um anzugeben, dass die verknüpfte Übung nicht als Teil des Übungsplans ausgewählt wurde;
b) Auswählens eines Teilsatzes von Übungen aus der Vielzahl von Übungen, um den Übungsplan zu erzeugen, und Aktualisierens des verknüpften Parameters jeder ausgewählten Übung in der Vielzahl von Übungen auf einen zweiten Wert, um anzugeben, dass die/jede ausgewählte Übung als Teil des Übungsplans ausgewählt wurde; und
c) nachdem der Benutzer den Übungsplan abschließt, Aktualisierens des Übungsplans auf Basis der Leistung des Benutzers, die sich aus der Ausführung des Übungsplans ergibt, und des Parameters, der mit jeder Übung aus der Vielzahl von Übungen verknüpft ist, wobei der Schritt c) umfasst:
c1) Löschen von mindestens einer Übung aus dem Übungsplan auf Basis der Leistung des Benutzers, die sich aus der Ausführung des Übungsplans ergibt, und Aktualisieren des Parameters jeder gelöschten Übung in der Vielzahl von Übungen auf einen dritten Wert, um anzugeben, dass die/jede gelöschte Übung vom Benutzer durchgeführt wurde; und
c2) Hinzufügen von mindestens einer Übung mit dem ersten Wert aus der Vielzahl von Übungen in den Übungsplan, und Aktualisieren des Parameters jeder hinzugefügten Übung in der Vielzahl von Übungen auf den zweiten Wert, und
wobei der Schritt c) weiter einen Schritt c4) nach dem Schritt c2) umfasst: Bestimmen, ob eine vordefinierte Bedingung erfüllt ist, und Aktualisieren des Parameters jeder der Übungen mit dem dritten Wert auf den ersten Wert, wenn die vordefinierte Bedingung erfüllt ist.

2. Verfahren nach Anspruch 1, wobei der Schritt c) weiter einen Schritt c3) vor dem Schritt c2) umfasst: Bestimmen, ob die Anzahl der Übungen mit dem ersten Wert größer als oder gleich der Differenz zwischen der Anzahl der Übungen im Teilsatz und der Anzahl der Übungen mit dem zweiten Wert ist.

3. Verfahren nach Anspruch 2, wobei das Hinzufügen von mindestens einer Übung mit dem ersten Wert aus der Vielzahl von Übungen in den Übungsplan im Schritt c2) umfasst:
wenn die Anzahl der Übungen mit dem ersten Wert größer als oder gleich der Differenz zwischen der Anzahl der Übungen im Teilsatz und der Anzahl der Übungen mit dem zweiten Wert ist, Hinzufügen von mindestens einer Übung mit dem ersten Wert aus der Vielzahl von Übungen in den Übungsplan, sodass die Summe aus der Anzahl der hinzugefügten Übung und der Anzahl der Übungen mit dem zweiten Wert gleich der Anzahl der Übungen im Teilsatz ist; oder
wenn die Anzahl der Übungen mit dem ersten Wert nicht größer als oder gleich der Differenz zwischen der Anzahl der Übungen im Teilsatz und der Anzahl der Übungen mit dem zweiten Wert ist, Hinzufügen aller der Übungen mit dem ersten Wert aus der Vielzahl von Übungen in den Übungsplan.

4. Verfahren nach Anspruch 1, wobei die vordefinierte Bedingung lautet, dass die Anzahl der Übungen mit dem ersten Wert kleiner als die Hälfte der Anzahl der Übungen im Teilsatz ist.

5. Verfahren nach Anspruch 1, weiter das Zurückkehren zum Schritt c1), nachdem der Schritt c4) durchgeführt ist, und mindestens einmalige Wiederholen der Schritte c1), c3), c2) und c4) umfassend.

6. Vorrichtung (100, 100', 100") zum Erzeugen eines Übungsplans aus einer Vielzahl von Übungen für einen Benutzer, wobei jede Übung aus der Vielzahl von Übungen mit einem Parameter verknüpft ist, um den Status der/jeder Übung aus der Vielzahl von Übungen anzugeben, wobei die Vorrichtung umfasst:
eine erste Einheit (101), die dazu konfiguriert ist, den Parameter jeder Übung aus der Vielzahl von Übungen mit einem ersten Wert zu initialisieren, um anzugeben, dass die verknüpfte Übung nicht als Teil des Übungsplans ausgewählt wurde;
eine zweite Einheit (102), die dazu konfiguriert ist, einen Teilsatz von Übungen aus der Vielzahl von Übungen, die initialisiert wurden, auszuwählen, um den Übungsplan zu erzeugen, und den verknüpften Parameter jeder ausgewählten Übung in der Vielzahl von Übungen auf einen zweiten Wert zu aktualisieren, um anzugeben, dass die/jede Übung im Teilsatz als Teil des Übungsplans ausgewählt wurde; und
eine dritte Einheit (103), die dazu konfiguriert ist, nachdem der Benutzer den Übungsplan abschließt, den Übungsplan auf Basis der Leistung des Benutzers, die sich aus der Ausführung des Übungsplans ergibt, und den Parameter, der mit jeder Übung aus der Vielzahl von Übungen verknüpft ist, zu aktualisieren, wobei die dritte Einheit umfasst:
eine erste Teileinheit (103a), die dazu konfiguriert ist, auf Basis der Leistung des Benutzers, die sich aus der Ausführung des Übungsplans ergibt, mindestens eine Übung aus dem Übungsplan zu löschen und den Parameter jeder gelöschten Übung in der Vielzahl von Übungen auf einen dritten Wert zu aktualisieren, um anzugeben, dass die/jede gelöschte Übung vom Benutzer durchgeführt wurde; und
eine zweite Teileinheit (103b), die dazu konfiguriert ist, mindestens eine Übung mit dem ersten Wert aus der Vielzahl von Übungen in den Übungsplan, aus dem mindestens eine Übung gelöscht wurde, hinzuzufügen und den Parameter jeder hinzugefügten Übung in der Vielzahl von Übungen auf den zweiten Wert zu aktualisieren, und
wobei die dritte Einheit (103) weiter eine vierte Teileinheit (103d) umfasst, die dazu konfiguriert ist, für die Vielzahl von Übungen, bei der der Parameter jeder hinzugefügten Übung, die in den Übungsplan hinzugefügt wurde, auf den zweiten Wert aktualisiert wurde, zu bestimmen, ob eine vordefinierte Bedingung erfüllt ist, und den Parameter jeder der Übungen mit dem dritten Wert auf den ersten Wert zu aktualisieren, wenn die vordefinierte Bedingung erfüllt ist.

7. Vorrichtung nach Anspruch 6, wobei die dritte Einheit weiter eine dritte Teileinheit (103c) umfasst, die dazu konfiguriert ist, für die Vielzahl von Übungen, bei der der Parameter jeder gelöschten Übung, die aus dem Übungsplan gelöscht wurde, auf den dritten Wert aktualisiert wurde, zu bestimmen, ob die Anzahl der Übungen mit dem ersten Wert größer als oder gleich der Differenz zwischen der Anzahl der Übungen im Teilsatz und der Anzahl der Übungen mit dem zweiten Wert ist.

8. Vorrichtung nach Anspruch 7, wobei die zweite Teileinheit (103b) weiter dazu konfiguriert ist:
wenn die Anzahl der Übungen mit dem ersten Wert größer als oder gleich der Differenz zwischen der Anzahl der Übungen im Teilsatz und der Anzahl der Übungen mit dem zweiten Wert ist, mindestens eine Übung mit dem ersten Wert aus der Vielzahl von Übungen in den Übungsplan, aus dem mindestens eine Übung gelöscht wurde, hinzuzufügen, sodass die Summe aus der Anzahl der hinzugefügten Übung und der Anzahl der Übungen mit dem zweiten Wert gleich der Anzahl der Übungen im Teilsatz ist; oder
wenn die Anzahl der Übungen mit dem ersten Wert nicht größer als oder gleich der Differenz zwischen der Anzahl der Übungen im Teilsatz und der Anzahl der Übungen mit dem zweiten Wert ist, alle die Übungen mit dem ersten Wert aus der Vielzahl von Übungen in den Übungsplan, aus dem mindestens eine Übung gelöscht wurde, hinzuzufügen.

9. Vorrichtung nach Anspruch 6, wobei die vordefinierte Bedingung lautet, dass die Anzahl der Übungen mit dem ersten Wert kleiner als die Hälfte der Anzahl der Übungen im Teilsatz ist.

10. Vorrichtung nach Anspruch 6, wobei die Operationen der ersten Teileinheit (103a), der dritten Teileinheit (103c), der zweiten Teileinheit (103b) und der vierten Teileinheit (103d) mindestens einmal wiederholt werden.

11. Informationsmedium, das dazu konfiguriert ist, ein Programm zu speichern, das von einem Prozessor in einem Computer geladen und ausgeführt wird, um ein Verfahren nach einem der Ansprüche 1 bis 5 durchzuführen.

## Revendications

1. Procédé implémenté par ordinateur pour la génération d'un plan d'exercice à partir d'une pluralité d'exercices pour un utilisateur, chaque exercice de la pluralité d'exercices étant associé à un paramètre pour indiquer le statut de chaque dit exercice de la pluralité d'exercices, le procédé comprenant les étapes de :
a) l'initialisation du paramètre de chaque exercice de la pluralité d'exercices dans une première valeur pour indiquer que l'exercice associé n'a pas été sélectionné comme faisant partie du plan d'exercice ;
b) la sélection d'un sous-ensemble d'exercices à partir de la pluralité d'exercices pour générer le plan d'exercice et la mise à jour du paramètre associé de chaque exercice sélectionné dans la pluralité d'exercices dans une deuxième valeur pour indiquer que chaque dit exercice sélectionné a été sélectionné comme faisant partie du plan d'exercice ; et
c) après que l'utilisateur finit de faire le plan d'exercice, la mise à jour du plan d'exercice sur la base de la performance d'utilisateur résultant de l'exécution du plan d'exercice et du paramètre associé à chaque exercice de la pluralité d'exercices, dans lequel l'étape c) comprend :
c1) la suppression d'au moins un exercice du plan d'exercice sur la base de la performance d'utilisateur résultant de l'exécution du plan d'exercice et la mise à jour du paramètre de chaque exercice supprimé dans la pluralité d'exercices dans une troisième valeur pour indiquer que chaque dit exercice supprimé a été réalisé par l'utilisateur ; et
c2) l'ajout d'au moins un exercice avec la première valeur de la pluralité d'exercices dans le plan d'exercice et la mise à jour du paramètre de chaque exercice ajouté dans la pluralité d'exercices dans la deuxième valeur, et
dans lequel l'étape c) comprend en outre une étape c4) après l'étape c2) : la détermination de la satisfaction d'une condition prédéfinie, et la mise à jour du paramètre de chacun des exercices avec la troisième valeur dans la première valeur si la condition prédéfinie est satisfaite.

2. Procédé selon la revendication 1, dans lequel l'étape c) comprend en outre une étape c3) avant l'étape c2) : la détermination si le nombre des exercices avec la première valeur est plus grand ou égal à la différence entre le nombre d'exercices dans le sous-ensemble et le nombre d'exercices avec la deuxième valeur.

3. Procédé selon la revendication 2, dans lequel l'ajout d'au moins un exercice avec la première valeur de la pluralité d'exercices dans le plan d'exercice dans l'étape c2) comprend :
si le nombre d'exercices avec la première valeur est plus grand ou égal à la différence entre le nombre d'exercices dans le sous-ensemble et le nombre d'exercices avec la deuxième valeur, l'ajout d'au moins un exercice avec la première valeur de la pluralité d'exercices dans le plan d'exercice de sorte que la somme du nombre de l'exercice ajouté et du nombre d'exercices avec la deuxième valeur soit égale au nombre d'exercices dans le sous-ensemble ; ou
si le nombre des exercices avec la première valeur n'est pas plus grand ou égal à la différence entre le nombre d'exercices dans le sous-ensemble et le nombre d'exercices avec la deuxième valeur, l'ajout de tous les exercices avec la première valeur de la pluralité d'exercices dans le plan d'exercice.

4. Procédé selon la revendication 1, dans lequel la condition prédéfinie est que le nombre d'exercices avec la première valeur est inférieur à la moitié du nombre d'exercices dans le sous-ensemble.

5. Procédé selon la revendication 1, comprenant en outre le retour à l'étape c1) après la réalisation de l'étape c4) et la répétition des étapes c1), c3), c2) et c4) au moins une fois.

6. Dispositif (100, 100', 100") pour la génération d'un plan d'exercice à partir d'une pluralité d'exercices pour un utilisateur, chaque exercice de la pluralité d'exercices étant associé à un paramètre pour indiquer le statut de chaque dit exercice de la pluralité d'exercices, le dispositif comprenant :
une première unité (101) configurée pour initialiser le paramètre de chaque exercice de la pluralité d'exercices dans une première valeur pour indiquer que l'exercice associé n'a pas été sélectionné comme faisant partie du plan d'exercice ;
une deuxième unité (102) configurée pour sélectionner un sous-ensemble d'exercices à partir de la pluralité d'exercices ayant été initialisés pour générer le plan d'exercice et mettre à jour le paramètre associé de chaque exercice sélectionné dans la pluralité d'exercices dans une deuxième valeur pour indiquer que chaque dit exercice dans le sous-ensemble a été sélectionné comme faisant partie du plan d'exercice ; et
une troisième unité (103) configurée pour, après que l'utilisateur finit de faire le plan d'exercice, mettre à jour le plan d'exercice sur la base de la performance d'utilisateur résultant de l'exécution du plan d'exercice et du paramètre associé à chaque exercice de la pluralité d'exercices,
dans lequel la troisième unité comprend :
une première sous-unité (103a) configurée pour supprimer au moins un exercice du plan d'exercice sur la base de la performance d'utilisateur résultant de l'exécution du plan d'exercice et mettre à jour le paramètre de chaque exercice supprimé dans la pluralité d'exercices dans une troisième valeur pour indiquer que chaque dit exercice supprimé a été réalisé par l'utilisateur ; et
une deuxième sous-unité (103b) configurée pour ajouter au moins un exercice avec la première valeur de la pluralité d'exercices dans le plan d'exercice duquel au moins un exercice a été supprimé et mettre à jour le paramètre de chaque exercice ajouté dans la pluralité d'exercices dans la deuxième valeur, et
dans lequel la troisième unité (103) comprend en outre une quatrième sous-unité (103d) configurée pour, pour la pluralité d'exercices dans laquelle le paramètre de chaque exercice ajouté dans le plan d'exercice a été mis à jour dans la deuxième valeur, déterminer si une condition prédéfinie est satisfaite et mettre à jour le paramètre de chacun des exercices avec la troisième valeur dans la première valeur si la condition prédéfinie est satisfaite.

7. Dispositif selon la revendication 6, dans lequel la troisième unité comprend en outre une troisième sous-unité (103c) configurée pour, pour la pluralité d'exercices dans lesquels le paramètre de chaque exercice supprimé du plan d'exercice a été mis à jour dans la troisième valeur, déterminer si le nombre d'exercices avec la première valeur est plus grand ou égal à la différence entre le nombre d'exercices dans le sous-ensemble et le nombre d'exercices avec la deuxième valeur.

8. Dispositif selon la revendication 7, dans lequel la seconde sous-unité (103b) est en outre configurée pour :
si le nombre d'exercices avec la première valeur est plus grand ou égal à la différence entre le nombre d'exercices dans le sous-ensemble et le nombre d'exercices avec la deuxième valeur, ajouter au moins un exercice avec la première valeur de la pluralité d'exercices dans le plan d'exercice duquel au moins un exercice a été supprimé de sorte que la somme du nombre de l'exercice ajouté et du nombre d'exercices avec la deuxième valeur soit égale au nombre d'exercices dans le sous-ensemble ; ou
si le nombre d'exercices avec la première valeur est plus grand ou égal à la différence entre le nombre d'exercices dans le sous-ensemble et le nombre d'exercices avec la deuxième valeur, ajouter tous les exercices avec la première valeur de la pluralité d'exercices dans le plan d'exercice duquel au moins un exercice a été supprimé.

9. Dispositif selon la revendication 6, dans lequel la condition prédéfinie est que le nombre d'exercices avec la première valeur est inférieur à la moitié du nombre d'exercices dans le sous-ensemble.

10. Dispositif selon la revendication 6, dans lequel les opérations de la première sous-unité (103a), la troisième sous-unité (103c), la deuxième sous-unité (103b) et la quatrième sous-unité (103d) sont répétées au moins une fois.

11. Support d'informations configuré pour stocker un programme chargé et exécuté par un processeur dans un ordinateur pour réaliser un procédé selon l'une quelconque des revendications 1 à 5.
